# EUROPÄISCHE PATENTSCHRIFT

(11) **EP 3 350 597 B1**
(45) Veröffentlichungstag und Bekanntmachung des Hinweises auf die Patenterteilung: **11.08.2021**
(21) Anmeldenummer: 16766311.1
(22) Anmeldetag: 16.09.2016
(51) Int. Cl.: G01N 33/543, B82Y 30/00, G01N 21/64

(54) **VERWENDUNG VON SUBSTRATEN ZUR FLUORESZENZVERSTÄRKUNG**
USE OF SUBSTRATES FOR FLUORESCENCE AMPLIFICATION
UTILISATION DE SUBSTRAT POUR L'AMPLICATION DE LA FLUORESCENCE

(30) Priorität: 16.09.2015 AT 507932015
(43) Veröffentlichungstag der Anmeldung: 25.07.2018
(73) Patentinhaber: Fianostics GmbH, 2700 Wiener Neustadt (AT); Stratec Consumables GmbH, 5081 Anif (AT)
(72) Erfinder: MAURACHER, Christoph, 5020 Salzburg (AT); BAUER, Georg, 5026 Salzburg-Aigen (AT); PRINZ, Adrian, Bad Reichenhall (DE); AICHINGER, Gottfried, 5400 Hallein (AT); HAWA, Gerhard, 1100 Wien (AT)
(74) Vertreter: Pföstl, Andreas
(86) Internationale Anmeldenummer: PCT/EP2016/071953
(87) Internationale Veröffentlichungsnummer: WO 2017/046320

(56) Entgegenhaltungen:
- WO-A1-2009/059204
- WO-A2-2007/094817
- US-A1- 2005 214 841
- US-A1- 2009 262 640
- LIU Y ET AL: "Biosensing based upon molecular confinement in metallic nanocavity arrays; Biosensing based upon molecular confinement in metallic nanocavity arrays", NANOTECHNOLOGY, IOP, BRISTOL, GB, Bd. 15, Nr. 9, 1. September 2004 (2004-09-01), Seiten 1368-1374, XP020068108, ISSN: 0957-4484, DOI: 10.1088/0957-4484/15/9/043
- CHOU S Y ET AL: "NONOIMPRINT LITHOGRAPHY", JOURNAL OF VACUUM SCIENCE AND TECHNOLOGY: PART B, AVS / AIP, MELVILLE, NEW YORK, NY, US, Bd. 14, Nr. 6, 1. November 1996 (1996-11-01), Seiten 4129-4133, XP000721138, ISSN: 1071-1023, DOI: 10.1116/1.588605 in der Anmeldung erwähnt
- LIU Y ET AL: "FLUORESCENCE ENHANCEMENT FROM AN ARRAY OF SUBWAVELENGTH METAL APERTURES", OPTICS LETTERS, OPTICAL SOCIETY OF AMERICA, US, Bd. 28, Nr. 7, 1. April 2003 (2003-04-01), Seiten 507-509, XP001160143, ISSN: 0146-9592, DOI: 10.1038/NATURE01916
- ALEXANDRE G. BROLO ET AL: "Enhanced Fluorescence from Arrays of Nanoholes in a Gold Film", JOURNAL OF THE AMERICAN CHEMICAL SOCIETY, Bd. 127, Nr. 42, 1. Oktober 2005 (2005-10-01), Seiten 14936-14941, XP055319569, US ISSN: 0002-7863, DOI: 10.1021/ja0548687
- THOMAS M. SCHMIDT ET AL: "Plasmonic Fluorescence Enhancement of DBMBF 2 Monomers and DBMBF 2 -Toluene Exciplexes using Al-Hole Arrays", JOURNAL OF PHYSICAL CHEMISTRY C, Bd. 118, Nr. 4, 30. Januar 2014 (2014-01-30), Seiten 2138-2145, XP055319573, US ISSN: 1932-7447, DOI: 10.1021/jp4110823
- Y. LIU ET AL: "Fluorescence Enhancement From a Periodic Array of Sub-Wavelength Metallic Cavities", INTEGRATED PHOTONICS RESEARCH: POSTCONFERENCE DIGEST; JUNE 15 - 20, 2003 (IN. TRENDS IN OPTICS AND PHOTONICS SERIES), 1. Januar 2003 (2003-01-01), Seite PD5, XP055319562, Washington, DC DOI: 10.1364/IPR.2003.PD5 ISBN: 978-1-55752-751-6
- FRANCES LORDAN ET AL: "Effect of Cavity Architecture on the Surface-Enhanced Emission from Site-Selective Nanostructured Cavity Arrays", JOURNAL OF PHYSICAL CHEMISTRY C, Bd. 116, Nr. 2, 19. Januar 2012 (2012-01-19), Seiten 1784-1788, XP055320115, US ISSN: 1932-7447, DOI: 10.1021/jp210343t
- Manas Ranjan Gartia ET AL: "Colorimetric Plasmon Resonance Imaging Using Nano Lycurgus Cup Arrays", Advanced Optical Materials, vol. 1, no. 1, 1 January 2013 (2013-01-01) , pages 68-76, XP055570582, DE ISSN: 2195-1071, DOI: 10.1002/adom.201200040
- Sujin Seo ET AL: "Colorimetric Effect of Gold Nanocup Arrays on Fluorescence Amplification", JOURNAL OF PHYSICAL CHEMISTRY C, vol. 119, no. 32, 3 August 2015 (2015-08-03), pages 18518-18526, XP055570568, ISSN: 1932-7447, DOI: 10.1021/acs.jpcc.5b03329

## Beschreibung

Die vorliegende Erfindung betrifft die Verwendung von nano-strukturierten Oberflächen, die sich in besonders vorteilhafter Weise dazu eignen, die Fluoreszenz geeigneter Moleküle bei Annäherung selbiger Moleküle an diese Oberflächen zu verstärken. Dieser Effekt ist auch als metallverstärkte Fluoreszenz (MEF, "Metal Enhanced Fluorescence") oder oberflächenverstärkte Fluoreszenz (SEF, "Surface Enhanced Fluorescence") bekannt.

MEF und SEF beruhen auf einer elektromagnetischen Interaktion des einfallenden (anregenden), meist kohärenten (d.h. Laser) Lichtes mit dem Elektronenplasma von Nano-Metallstrukturen. Dies führt zu einer Verstärkung der Lichtausbeute fluoreszierender Moleküle bei deren Annäherung (z.B. Bindung) an eine Oberfläche, die derartige Metallstrukturen aufweist. Dadurch leuchten an der Oberfläche gebundene Moleküle intensiver, weil ihre Fluoreszenz verstärkt wird.

Durch die Verstärkung der Fluoreszenz können an eine Oberfläche gebundene Moleküle in geringsten Konzentrationen gemessen werden. Beispielsweise lässt sich die Bindung eines fluoreszenzmarkierten Antikörpers in Form seiner Bindungskinetik direkt verfolgen.

Das Ausmaß der Verstärkung ist von Form, Größe und Abstand der Nano-Metallstrukturen und vom der verwendeten Metallart (z.B. Au, Ag, Al etc.) abhängig. So finden sich in der Literatur Beschreibungen von sphärischen (häufig Kolloide; siehe z.B. B Yang et al. Small 6(2010): 1038-43; Corrigan T et al J Fluorescence 15(2005):777-784) dreieckigen bzw. pyramidenförmigen (siehe z.B. Pompa et al. Nature Nanotechnology 1(2006):126 - 130; Cade et al. Nanotechnology. 15(2009):20(28)) oder draht- bzw. stabförmigen Metallstrukturen, die nicht zusammenhängend sind und sogenannte Metallinseln ausbilden. Die erzielten Verstärkungsfaktoren schwanken jedoch stark und die Nano-Metallstrukturen sind in den meisten Fällen nicht reproduzierbar.

In der US 2005/214841 werden Substrate beschrieben, welche eine Vielzahl von Vertiefungen aufweisen und zumindest teilweise mit einem Metall beschichtet sind. Das Substrat kann aus verschiedensten Materialien wie z.B. Glas, Keramik oder Metall bestehen. Auf die Oberfläche des Substrats gemäß der US 2005/214841, insbesondere in dessen Vertiefungen, werden Linker aufgebracht, die aufgrund funktioneller Gruppen in der Lage sind, biologische Substanzen zu immobilisieren. In dieser US Anmeldung wird nicht erwähnt, dass die darin beschriebenen Substrate geeignet wären, die Fluoreszenz von Molekülen zu verstärken.

In der US 6,902,705 werden Substrate beschrieben, welche ebenfalls Vertiefungen umfassen und mit einem Metall (z.B. Gold) beschichtet sein können. Die Oberfläche der Substrate wird modifiziert, um die Bindung von biologischen Substanzen (z.B. DNA) an der Oberfläche des Substrats zu ermöglichen. Das Vorsehen von Vertiefungen am Substrat scheint den Vorteil zu haben, dass dadurch Fluoreszenzmessungen durchgeführt werden können, ohne störende und interferierende Signale zu erhalten. Eine Verstärkung des Fluoreszenzsignals kann jedoch mit diesen Substraten nicht erzielt werden.

In der WO 2009/059204 werden Substrate geoffenbart, die zur Verstärkung der Fluoreszenz eines oder mehrerer fluoreszierender Moleküle eingesetzt werden können. Die Oberfläche dieser Substrate kann Vertiefungen aufweisen, in denen die zu detektierenden Moleküle eingebracht werden können.

In der WO 2007/094817 werden Elemente beschrieben, mit denen die Fluoreszenz fluoreszierender Moleküle verstärkt werden kann und die ein mit einer Metallschicht überzogenes Substrat umfassen. Die metallische Schicht weist zumindest eine Vertiefung und eine Dicke von ca. 100 nm auf. Die in diesem Dokument offenbarten Vertiefungen geoffenbart haben einen Durchmesser von ca. 200 nm und eine Tiefe von 150 nm.

In Liu Y et al. (Nanotechn 15(2004):1368-1374) werden Biosensoren beschrieben, die an deren Oberfläche Vertiefungen aufweisen und in der Lage sind die Fluoreszenz von fluoreszierenden Molekülen an deren Oberfläche zu verstärken Der Durchmesser dieser Vertiefungen wird mit ca. 200 nm, der Abstand der Vertiefungen zueinander mit ca. 1 µm angegeben. Vergleichbar mit Liu Y et al. werden auch in Liu Y et al. (Opt Lett 28(2003):507-509), Brolo AG et al. (J Am Chem Soc 127(2005):14936-14941) und Schmidt TM et al. (J Phys Chem 118(2014):2138-2145) vergleichbare Substrate angeführt.

In Gartia MR et al. (Adv Optical Mater 1(2013):68-76) werden Arrays offenbart, an deren Oberfläche "Nano-Lycurgus-Kelche" ("Nano Lycurgus Cups") angeordnet sind. Diese Arrays können für kolorimetrische bildgebende Plasmonenresonanz ("colorimetric plasmon resonance imaging") eingesetzt werden. In der später veröffentlichten Publikation Seo S et al. (J Phys Chem C 119(2015):18518-18526) wird festgehalten, dass die in Gartia MR et al. beschriebenen Substrate zur Verstärkung von Fluoreszenz herangezogen werden können.

Die vorliegende Erfindung betrifft die Verwendung eines Substrats zur Verstärkung der Fluoreszenz eines oder mehrerer fluoreszierender Moleküle, wobei das Substrat einen festen polymeren Träger mit einer Mehrzahl voneinander getrennter Vertiefungen umfasst und der feste Träger zumindest teilweise mit Silber oder Legierungen umfassend Silber beschichtet ist, wobei die Vertiefungen einen Abstand zueinander von 0,2 µm bis 2,5 µm und eine Tiefe von 0,1 µm bis 5 µm aufweisen und die Metallschicht am festen Träger eine Dicke von 10 nm bis 60 nm aufweist.

Es hat sich überraschender Weise herausgestellt, dass Substrate mit dem erfindungsgemäßen Aufbau in der Lage sind, die Fluoreszenzausbeute (Quantenausbeute) eines fluoreszierenden Moleküls bzw. eines Fluorophors mit und ohne Verwendung kohärenten Lichtes signifikant zu steigern sofern sich das mindestens eine fluoreszierende Molekül bzw. Fluorophor in der Nähe befindet (metallverstärkte Fluoreszenz; MEF). Unter "Fluoreszenzausbeute" bzw. "Quantenausbeute" wird das Verhältnis zwischen der Anzahl der emittierten und absorbierten Photonen verstanden.

Die Fluoreszenzausbeute mit den Substraten ist sogar um ein Vielfaches höher als die Ausbeute unter Verwendung bisher bekannter Substrate, an deren Oberfläche sich in der Regel metallische Inseln befinden. Diese Steigerung in der Fluoreszenzausbeute ist dahingehend überraschend, da man bisher davon ausging, dass der MEF-Effekt nur an Oberflächen zustande kommen kann, die metallische Inseln in Form von abgelagerten Metall-haltigen Kolloiden oder sonstigen voneinander isolierter und Metall-beschichteter Bereiche auf einer Oberfläche aufweisen (Matveeva E. et al., Anal Biochem _334(2004):303-11; Geddes C.D., et al. J Fluoresc 12(2002):121-129). Substrate mit einer durchgehenden Metallschicht bzw. ohne Erhebungen sind bekannt dafür wegen eines Fluoreszenz-Quenching Effekts der Metalloberfläche selbst, keinen oder einen sehr geringen MEF-Effekt zu zeigen (Pineda E.C., et al. J. Chem. Phys. 83(1985):5330-5337; Barnes W.L., J Mod Opt, 45(1998):661-699). Aus diesem Grund hätte ein Fachmann - ohne Kenntnis der vorliegenden Erfindung - einen festen Träger mit Erhebungen und nicht einen Träger mit Vertiefungen zum Beschichten mit einem Metall ausgewählt. Erfindungsgemäß wird das Substrat zur Verstärkung der Fluoreszenz von Fluorophoren eingesetzt. D.h. erfindungsgemäß finden die Substrate überall dort Anwendung wo die Verstärkung der Fluoreszenz (d.h. eine Erhöhung der Fluoreszenzausbeute) erwünscht ist. Daher kann das Substrat z.B. bei Immunoassays, jeder Form der molekularen Diagnostik mittels Nukleinsäuren (PCR, RT-PCR), zellbasierten Bioassays (wie sie häufig beim High-Throughput-Screening (Hochdurchsatz-Screening) vorkommen, histologischen oder zellulären Untersuchungen, Multi-Plexing Testsystemen (z.B. LUMINEX) verwendet werden, sofern Fluoreszenz zum Nachweis der Zielmoleküle eingesetzt wird.

Gemäß einer bevorzugten Ausführungsform der vorliegenden Erfindung erfolgt die Verstärkung der Fluoreszenz in einem Abstand von 0 bis 50 nm, vorzugsweise von 1 bis 50 nm, noch mehr bevorzugt von 1 bis 40 nm, noch mehr bevorzugt von 2 bis 40 nm, noch mehr bevorzugt von 1 bis 30 nm, noch mehr bevorzugt von 2 bis 30 nm, noch mehr bevorzugt von 3 bis 30 nm, noch mehr bevorzugt von 1 bis 20 nm, noch mehr bevorzugt von 2 bis 20 nm, noch mehr bevorzugt von 3 bis 20 nm, noch mehr bevorzugt von 5 bis 20 nm, noch mehr bevorzugt von 5 bis 15 nm, zu dem Metall, welches sich auf der Oberfläche des festen polymeren Trägers befindet.

Unter "fluoreszierende Moleküle", wie hier verwendet, werden erfindungsgemäß Moleküle subsummiert, welche nach Anregung durch elektromagnetische Wellen, wie z.B. Licht einer bestimmten Wellenlänge, spontan Licht emittieren. "Fluorophore" ist hierbei ein Überbegriff und ein Synonym solcher Moleküle und umfasst somit auch Moleküle, die fluoreszieren bzw. schwach fluoreszieren und meist nicht als Fluorophore bezeichnet werden. Beispiele solcher Moleküle sind Proteine oder Nukleinsäuren, deren Fluoreszenz ("intrinsische Fluoreszenz") über aromatische Strukturen (z.B. über die Aminosäuren Tryptophan oder Tyrosin) vermittelt wird.

Der "feste Träger" kann erfindungsgemäß aus einem beliebigen polymeren Material bestehen, sofern sich dieses mit einem Metall beschichten lässt und sofern Vertiefungen erzeugt werden können. Beispielsweise umfasst oder besteht der feste polymere Träger aus synthetischen Polymeren wie Polystyrol, Polyvinylchlorid oder Polykarbonat, Cyloolefin, Polymethylemthacrylat, Polylaktat oder Kombinationen davon. Grundsätzlich wären auch nichtpolymere Träger wie Metalle, Keramiken oder auch Glas einsetzbar, sofern sich dieser mit einem Metall beschichten lässt und sofern Vertiefungen erzeugt werden können.

Der feste Träger umfasst mindestens ein Material ausgewählt aus der Gruppe bestehend aus der Gruppe der thermoplastischen Polymere und der Polykondensate.

Gemäß einer bevorzugten Ausführungsform der vorliegenden Erfindung ist das thermoplastische Polymer ausgewählt aus der Gruppe bestehend aus Polyolefine, Vinylpolymere, Styrolpolymere, Polyacrylate, Polyvinylcarbazol, Polyacetal und Fluorkunstoffe.

Das Polykondensat ist vorzugsweise ausgewählt aus der Gruppe bestehend aus thermoplastische Polykondensate, duroplastische Polykondensate und Polyaddukte.

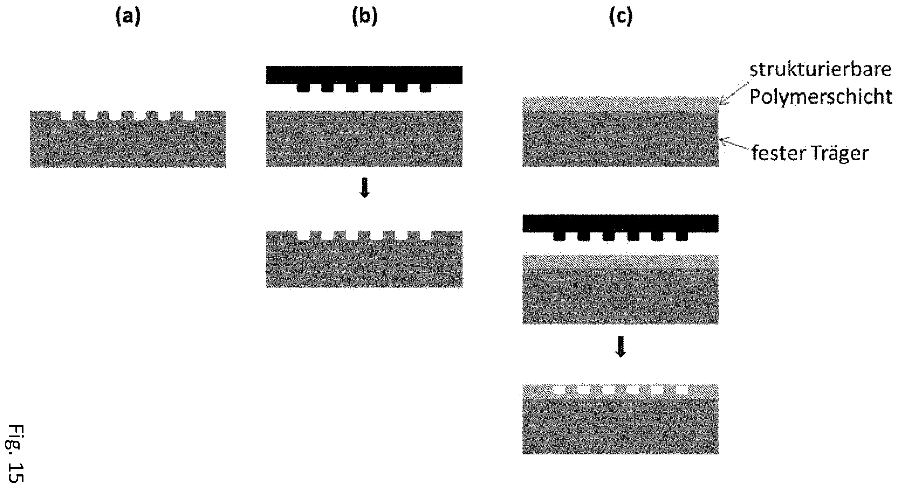

Gemäß einer besonders bevorzugten Ausführungsform der vorliegenden Erfindung umfasst das Material des polymeren festen Trägers organische und/oder anorganische Zusatzstoffe und/oder Füllstoffe, wobei diese vorzugsweise ausgewählt sind aus der Gruppe bestehend aus TiO₂, Glas, Kohlenstoff, Farbpigmente, Lipide und Wachse. Ein Substrat zur Verstärkung der Fluoreszenz eines Fluorophors ist nach einem Verfahren gemäß der vorliegenden Offenbarung beschrieben herstellbar. Die festen Träger inklusive Vertiefungen können grundsätzlich mit verschiedenen Verfahren hergestellt werden (siehe Fig. 15).
(a) Die festen Träger werden mit samt den Vertiefungen in einem Schritt gefertigt (z.B. Spritzguss) (siehe Fig. 15(a))
(b) Die Vertiefungen werden in einen bestehenden festen Träger in weiteren Prozessschritten einbracht (z.B. Heißprägen, Elektronenstrahllithografie oder "Extreme Ultra Violet" (EUV) in Verbindung mit Reaktiven Ionenätzen oder Laserablation) (siehe Fig. 15(b))
(c) Auf einem festen Träger wird eine dünne strukturierbare Polymerschicht aufgebracht, in welche die Vertiefungen eingebracht werden, wie beispielsweise bei der Herstellung der BD-50 Blu-ray Disc (UV-Nanoimprintlithographie) (siehe Fig. 15(c)).

Besonders geeignet zur Herstellung dieser Strukturen ist auch die sogenannte Nanoprägelithografie (Chou S. et al., Nanoimprint lithography, Journal of Vacuum Science & Technology B Volume 14, Nr. 6, 1996, S. 4129-4133) angewandt werden. Zur Herstellung von Nanostrukturen mittels Nanoprägelithografie benötigt man ein Positiv, meist ein Monomer oder Polymer, sowie einen nanostrukturierten Stempel ("Master"). Der Stempel selbst kann durch Nanolithographie produziert werden, wobei dieser alternativ auch durch Ätzen hergestellt werden kann. Das Positiv wird auf ein Substrat aufgebracht und anschließend über die Temperatur des Glasübergangs erhitzt, d.h., es wird verflüssigt, bevor der Stempel eingedrückt wird. Um ein kontrollierbares (und kurzzeitiges) Aufheizen zu erreichen, wird häufig Laser bzw. UV-Licht eingesetzt. Auf Grund der Viskosität des Positivs beim Erhitzen werden die Zwischenräume des Stempels vollständig damit ausgefüllt. Nach dem Abkühlen wird der Stempel wieder entfernt. Das Positiv, das den festen Träger des Substrats darstellt, wird mittels Sputter-Verfahren mit Metall beschichtet.

Die Strukturierung der Stempel für die Lithografie kann wiederum mit Nanoimprint erfolgen. Als Materialien wird dabei Glas oder lichttransparenter Kunststoff verwendet.

Besonders bevorzugt ist die Herstellung des festen Trägers inklusive Vertiefungen mittels Spritzguss. Die Formeinsätze hierfür werden typischerweise mittels Ni-Galvanik von einem lithografisch hergestellten Si-Wafer abgezogen

Der feste Träger kann grundsätzlich eine beliebige Form (z.B. kugelförmig, planar) aufweisen, wobei eine planare Form besonders bevorzugt ist.

Eine "Vertiefung", wie hier verwendet, bezieht sich auf das Niveau der die Vertiefung umgebenden Oberfläche des festen Trägers und erstreckt sich in den Träger hinein und nicht wie eine Erhebung oder Erhöhung aus diesem heraus. Eine Vertiefung im Sinne der vorliegenden Erfindung weist einen Boden auf, der durch die Seitenwände begrenzt ist. Die Tiefe ist somit der Abstand von der Oberfläche bis zu einem Boden der Vertiefung. Die Vertiefungen am festen Träger können unterschiedliche Formen (z.B. rund, oval, viereckig, rechteckig) aufweisen.

Eine "Mehrzahl" von Vertiefungen, wie hier verwendet, bedeutet, dass der feste Träger mindestens eine, vorzugsweise mindestens zwei, noch mehr bevorzugt mindestens 5, noch mehr bevorzugt mindestens 10, noch mehr bevorzugt mindestens 20, noch mehr bevorzugt mindestens 30, noch mehr bevorzugt mindestens 50, noch mehr bevorzugt mindestens 100, noch mehr bevorzugt mindestens 150, noch mehr bevorzugt mindestens 200, Vertiefungen aufweist. Diese Vertiefungen können auf einer Fläche des festen Trägers von 1000 µm², vorzugsweise von 500 µm², noch mehr bevorzugt von 200 µm², noch mehr bevorzugt von 100 µm², vorgesehen sein. Alternativ können sich die Vertiefungen über eine Länge von vorzugsweise 1000 pm, noch mehr bevorzugt von 500 µm, noch mehr bevorzugt von 200 µm, noch mehr bevorzugt von 100 µm, erstrecken.

"Voneinander getrennte Vertiefungen", wie hier verwendet, bedeutet, dass die Vertiefungen durch deren Seitenbegrenzungen voneinander getrennt und keine Verbindung zueinander aufweisen auch nicht an der Oberfläche des festen Trägers.

Gemäß einer bevorzugten Ausführungsform der vorliegenden Erfindung weisen die Vertiefungen des festen Trägers eine Länge und eine Breite aufweisen, wobei das Verhältnis der Länge zur Breite 2:1 bis 1:2, insbesondere ca. 1:1, beträgt.

Die Vertiefungen am festen Träger können im Prinzip jede Form aufweisen. Besonders bevorzugt sind jedoch Vertiefungen, die ein Verhältnis der Länge zur Breite von 2:1 bis 1:2, vorzugsweise 1,8:1, vorzugsweise 1,6:1, vorzugsweise 1,5:1, vorzugsweise 1,4:1, vorzugsweise 1,3:1, vorzugsweise 1,2:1, vorzugsweise 1,1:1, vorzugsweise 1:1,8, vorzugsweise 1:1,6, vorzugsweise 1:1,5, vorzugsweise 1:1,4, vorzugsweise 1:1,3, vorzugsweise 1:1,2, vorzugsweise 1:1,1, insbesondere 1:1, aufweisen.

Gemäß einer weiteren bevorzugten Ausführungsform der vorliegenden Erfindung beträgt die Länge und die Breite der Vertiefungen 0,1 µm bis 2 µm, vorzugsweise 0,2 µm bis 2 µm, vorzugsweise 0,3 µm bis 2 µm, vorzugsweise 0,1 µm bis 1,8 µm, vorzugsweise 0,2 µm bis 1,8 µm, vorzugsweise 0,3 µm bis 1,8 µm, vorzugsweise 0,1 µm bis 1,5 µm, vorzugsweise 0,2 µm bis 1,5 µm, vorzugsweise 0,3 µm bis 1,5 µm, vorzugsweise 0,1 µm bis 1,2 µm, vorzugsweise 0,2 µm bis 1,2 µm, vorzugsweise 0,2 µm bis 1,2 µm, vorzugsweise 0,1 µm bis 1 µm, vorzugsweise 0,2 µm bis 1 µm, vorzugsweise 0,3 µm bis 1 µm, vorzugsweise 0,1 µm bis 0,8 µm, vorzugsweise 0,2 µm bis 0,8 µm, vorzugsweise 0,3 µm bis 0,8 µm, vorzugsweise 0,1 µm bis 0,6 µm, vorzugsweise 0,2 µm bis 0,6 µm, vorzugsweise 0,3 µm bis 0,6 µm, am meisten bevorzugt 0,2 µm bis 0,6 µm.

Besonders bevorzugt weisen die Vertiefungen des festen Trägers eine im Wesentlichen runde Form auf, wobei "im Wesentlichen rund" auch ovale und ellipsoide Formen einschließt. Die Form der Vertiefung ist bei einer Draufsicht auf die Oberfläche des festen Trägers erkennbar.

Die Vertiefungen weisen eine Tiefe von 0,1 µm bis 5 µm, vorzugsweise von 0,1 µm bis 4 µm, vorzugsweise von 0,1µm bis 3 µm, vorzugsweise von 0,1 pm bis 2 pm, vorzugsweise von 0,1 pm bis 1,5 pm, vorzugsweise von 0,1 pm bis 1,2 pm, vorzugsweise von 0,1 pm bis 1 pm, vorzugsweise von 0,1 pm bis 0,9 pm, vorzugsweise von 0,1 pm bis 0,8 pm, vorzugsweise von 0,2 pm bis 5 pm, vorzugsweise von 0,2 pm bis 4 pm, vorzugsweise von 0,2 pm bis 3 pm, vorzugsweise von 0,2 pm bis 2 pm, vorzugsweise von 0,2 pm bis 1,5 pm, vorzugsweise von 0,2 pm bis 1,2 pm, vorzugsweise von 0,2 pm bis 1 pm, vorzugsweise von 0,2 pm bis 0,9 pm, vorzugsweise von 0,2 pm bis 0,8 pm, vorzugsweise von 0,3 pm bis 5 pm, vorzugsweise von 0,3 pm bis 4 pm, vorzugsweise von 0,3 pm bis 3 pm, vorzugsweise von 0,3 pm bis 2 pm, vorzugsweise von 0,3 pm bis 1,5 pm, vorzugsweise von 0,3 pm bis 1,2 pm, vorzugsweise von 0,3 pm bis 1 pm, vorzugsweise von 0,3 pm bis 0,9 pm, vorzugsweise von 0,3 pm bis 0,8 pm, auf. Die Tiefe der Vertiefung ist der Abstand von der Oberfläche des festen mit Metall bedampften Trägers bis zu dem Boden der Vertiefung.

Gemäß einer bevorzugten Ausführungsform der vorliegenden Erfindung weisen die Vertiefungen einen Abstand ("Periode") zueinander von 0,3 pm bis 1,4 pm, vorzugsweise von 0,4 pm bis 1,3 pm, auf. In einer weiteren bevorzugten Ausführungsform der vorliegenden Erfindung wiesen die Vertiefungen einen Abstand zueinander von 0,2 pm bis 2 pm, vorzugsweise von 0,2 pm bis 1,8 pm, vorzugsweise von 0,2 pm bis 1,6 pm, vorzugsweise von 0,2 pm bis 1,5 pm, vorzugsweise von 0,2 pm bis 1,4 pm, vorzugsweise von 0,2 pm bis 1,3 pm, vorzugsweise von 0,3 pm bis 2,5 pm, vorzugsweise von 0,3 pm bis 2 pm, vorzugsweise von 0,3 pm bis 1,8 pm, vorzugsweise von 0,3 pm bis 1,6 pm, vorzugsweise von 0,3 pm bis 1,5 pm, vorzugsweise von 0,3 pm bis 1,3 pm, vorzugsweise von 0,4 pm bis 2,5 pm, vorzugsweise von 0,4 pm bis 2 pm, vorzugsweise von 0,4 pm bis 1,8 pm, vorzugsweise von 0,4 pm bis 1,6 pm, vorzugsweise von 0,4 pm bis 1,5 pm, vorzugsweise von 0,4 pm bis 1,4, vorzugsweise von 0,5 pm bis 2,5 pm, vorzugsweise von 0,5 pm bis 2 pm, vorzugsweise von 0,5 pm bis 1,8 pm, vorzugsweise von 0,5 pm bis 1,6 pm, vorzugsweise von 0,5 pm bis 1,5 pm, vorzugsweise von 0,5 pm bis 1,4 pm, vorzugsweise von 0,5 pm bis 1,3 pm, vorzugsweise von 0,6 pm bis 2,5 pm, vorzugsweise von 0,6 pm bis 2 pm, vorzugsweise von 0,6 pm bis 1,8 pm, vorzugsweise von 0,6 pm bis 1,6 pm, vorzugsweise von 0,6 pm bis 1,5 pm, vorzugsweise von 0,6 pm bis 1,4 pm, vorzugsweise von 0,6 µm bis 1,3 pm, vorzugsweise von 0,7 µm bis 2,5 pm, vorzugsweise von 0,7 im bis 2 µm, vorzugsweise von 0,5 im bis 1,8 pm, vorzugsweise von 0,7 pm bis 1,6 µm, vorzugsweise von 0,7 pm bis 1,5 µm, vorzugsweise von 0,7 im bis 1,4 pm, vorzugsweise von 0,7 µm bis 1,3 pm, auf, wobei die Vertiefungen am meisten bevorzugt einen Abstand von 0,2 µm bis 1,4 pm bzw. 0,3 µm bis 1,3 µm zueinander aufweisen. Der Abstand zwischen den Vertiefungen ("Periode") wird vom Mittelpunkt der Vertiefung gemessen.

Gemäß einer weiteren bevorzugten Ausführungsform der vorliegenden Erfindung weist die Metallschicht am festen Träger eine Dicke von von 10 nm bis 50 nm, vorzugsweise von 15 nm bis 60 nm, vorzugsweise von 15 nm bis 50 nm, vorzugsweise von 20 nm bis 60 nm, vorzugsweise von 20 nm bis 50 nm. Erfindungsgemäß ist der feste polymere Träger "zumindest teilweise" mit mindestens einem Metall beschichtet. "Zumindest teilweise", wie hier verwendet, bedeutet, dass jener Bereich des festen Trägers, in dem sich die Vertiefungen befinden zumindest zu 20%, vorzugsweise zumindest zu 30%, noch mehr bevorzugt zumindest zu 40%, noch mehr bevorzugt zumindest zu 50%, noch mehr bevorzugt zumindest zu 60%, noch mehr bevorzugt zumindest zu 70%, noch mehr bevorzugt zumindest zu 80%, noch mehr bevorzugt zumindest zu 90%, noch mehr bevorzugt zumindest zu 95%, noch mehr bevorzugt zumindest zu 98%, insbesondere zu 100%, mit Silber oder Legierungen umfassend Silber beschichtet ist. Da der MEF-Effekt eine metallische Oberfläche voraussetzt, ist es besonders bevorzugt, dass die Oberfläche des festen Trägers zumindest im Bereich der Vertiefungen mit mindestens einem Metall beschichtet ist. Dabei kann der feste Träger auch mehrere (z.B. mindestens zwei, mindestens drei, mindestens vier oder mindestens fünf) übereinander angeordnete Metallschichten aus demselben oder unterschiedlichen Metallen umfassen. Ein Vorteil für die Verwendung mehrerer Schichten an Metall am festen Träger ist, dass die erste Metallschicht (z.B. Chrom), die direkt auf den Träger aufgebracht wird, die Haftung der weiteren Metallschichten verbessern kann.

Der Begriff "übereinander angeordnet", wie hierin verwendet, bedeutet, dass eine Metallschicht mittelbar oder unmittelbar auf einer anderen Metallschicht angeordnet ist. Dadurch ergibt sich ein mehrschichtiges System aus Metallschichten des gleichen Metalls oder unterschiedlicher Metalle.

Die Metallschichten sind vorzugsweise kontinuierlich und nicht unterbrochen. Erfindungsgemäß konnte jedoch festgestellt werden, dass die Metallschicht oder Metallschichten am festen polymeren Träger auch unterbrochen sein können, ohne dass der Fluoreszenz-verstärkende Effekt beeinträchtigt wird. Die unterbrochene Metallschicht kann beispielsweise durch eine Leitfähigkeitsmessung der Oberfläche des erfindungsgemäßen Substrats erfolgen. Eine geringere bzw. keine Leitfähigkeit bedeutet, dass die Metallschicht(en) an der Substratoberfläche unterbrochen sind. Unterbrochene Metallschichten können beispielsweise dadurch hergestellt werden, in dem ein im Wesentlichen vollständig mit Metall beschichtetes Substrat mit einer vorzugsweise salzhaltigen Lösung, wie beispielsweise 10mM Phosphatpuffer mit 150mM NaCl für einen bestimmten Zeitraum (10-90 Minuten) in Kontakt gebracht wird.

Der feste Träger ist mindestens mit Silber oder oder einer Legierung umfassend Silber beschichtet. Vorzugsweise umfasst die Metallschicht mindestens zwei, noch mehr bevorzugt mindestens drei, noch mehr bevorzugt mindestens vier, noch mehr bevorzugt mindestens fünf, verschiedene Metalle. Die Metalle können mit im Stand der Technik bekannte Methoden auf den festen Träger aufgebracht werden, wobei vorzugsweise Sputtern (Kathodenzerstäubung) oder thermisches Verdampfen, Elektronenstrahlverdampfen, Laserstrahlverdampfen, Lichtbogenverdampfen, Molekularstrahlepitaxie, Ionenstrahl gestützte Deposition und Ionenplattieren eingesetzt wird.

Das zusätzliche Metall kann ausgewählt sein aus der Gruppe bestehend aus Gold, Aluminium, Chrom, Indium, Kupfer, Nickel, Palladium, Platin, Zink, Zinn und Legierungen umfassend eines oder mehrerer dieser Metalle.

Diese Metalle bzw. Legierungen davon können zum Beschichten des festen Trägers verwendet werden. Erfindungsgemäß ist die Beschichtung des festen Trägers mit Silber oder Legierungen umfassend Silber, da Silber bzw. dessen Legierungen einen besonders großen Verstärkungseffekt zeigen. Besonders bevorzugt ist eine Legierung, die Silber, Indium und Zinn umfasst. Die Silber-haltigen Legierungen weisen vorzugsweise einen Silbergehalt von mehr als 10%, noch mehr bevorzugt von mehr als 30%, noch mehr bevorzugt von mehr als 50%, noch mehr bevorzugt von mehr als 70%, noch mehr bevorzugt von mehr als 80%, noch mehr bevorzugt von mehr als 90%, auf.

Nach dem Beschichten des festen Trägers mit mindestens einem Metall oder vor Verwendung des Substrats bzw. des festen Trägers, wird der feste Träger bzw. das Substrat vorzugsweise mit einer wässrigen Zusammensetzung umfassend mindestens eine Säure oder ein Salz eines Halogens ausgewählt aus der Gruppe bestehend aus Fluor, Chlor, Brom und Iod behandelt.

Es hat sich gezeigt, dass die Fluoreszenzverstärkung durch die Vorbehandlung des Substrats bzw. des festen Trägers mit einer wässrigen Lösung (z.B. einem Puffer) umfassend mindestens eine Säure eines Halogens oder seines Salzes davon nochmals verstärkt werden kann. Daher ist es besonders bevorzugt den festen Träger bzw. das Substrat mit einer säure- bzw. salzhaltigen Lösung vorzubehandeln.

Alternativ dazu kann die wässrige Lösung (z.B. einem Puffer) umfassend mindestens eine Säure oder ein Salz eines Halogens anstelle anderer Lösungen auch während der Messung eingesetzt werden.

Erfindungsgemäß eignen sich sämtliche Säuren der Halogengruppe oder deren Salze , wobei jedoch die radioaktiven Halogene in der Praxis nicht erwünscht werden. Daher werden besonders bevorzugt die Säuren oder Salze der Halogene Fluor, Chlor, Brom und Iod, am meisten bevorzugt Chloride, insbesondere Metallchloride, verwendet. Die erfindungsgemäß eingesetzten Säuren oder Salze sind besonders bevorzugt Alkalimetall- oder Erdalkalimetallsalze, insbesondere Natrium-, Kalium- oder Lithiumsalze.

Gemäß einer besonders bevorzugten Ausführungsform der vorliegenden Erfindung umfasst die wässrige Zusammensetzung mindestens eine Säure oder ein Salz ausgewählt aus der Gruppe bestehend aus HCl, HF, HBr, HJ, NaCl, NaF, NaBr, NaJ, KCl, KF, KBr und KJ.

Die wässrige Zusammensetzung umfassend mindestens einer Säure eines Halogens oder dessen Salzes kann neben der mindestens einen Säure oder ihres Salzes weitere Stoffe wie z.B. andere Säuren oder Salze umfassen. Besonders bevorzugt werden Stoffe, welche eine Pufferfunktion erfüllen, eingesetzt (z.B. Dinatriumhydrogenphosphat, Kaliumdihydrogenphosphat, Carbonate).

Gemäß einer weiteren bevorzugten Ausführungsform der vorliegenden Erfindung wird der feste Träger mit der wässrigen Zusammensetzung für mindestens 1, vorzugsweise für mindestens 2, noch mehr bevorzugt für mindestens 5, noch mehr bevorzugt für mindestens 10, noch mehr bevorzugt für mindestens 20, Minuten behandelt.

Es hat sich erfindungsgemäß gezeigt, dass der Fluoreszenz-verstärkende Effekt des mit mindestens mit Silber oder einer Legierung umfassend Silber beschichteten Trägers besonders hoch ist, wenn der feste Träger für mindestens 1 Minute mit der wässrigen Zusammensetzung umfassend mindestens eine Säure eines Halogens oder seines Salzes, vorzugsweise bei Raumtemperatur (22°C), inkubiert wird. Erfolgt die Inkubation bei höheren Temperaturen (z.B. zwischen 30°C und 40°C) kann die Inkubationszeit entsprechend reduziert werden (z.B. mindestens 30 Sekunden). Erfolgt die Inkubation jedoch bei tieferen Temperaturen (z.B. zwischen 10°C und 20°C) kann die Inkubationszeit entsprechend verlängert werden (z.B. mindestens 2 Minuten).

Gemäß einer bevorzugten Ausführungsform ist das Substrat Teil einer Kapillare, einer Mikrotiterplatte, eines mikrofluidischen Chips, eines Teststreifens (für "Lateral Flow Assays"), eines Trägers (z.B. Objektträgers) für die Fluoreszenzmikroskopie, insbesondere für hochauflösende Verfahren wie die konfokale Lasermikroskopie nach dem Punktscanner-Prinzip sowie 4Pi-Mikroskope und STED (Stimulated Emission Depletion)-Mikroskope, eines Sensor Arrays oder sonstigem optischen Detektorfeldes ist.

Besonders bevorzugt ist die Verwendung des Substrats in Mikrotiterplatten, wobei die Mikrotiterplatten 6, 12, 24, 48, 96, 384 oder 1536 Wells umfassen können. Mikrotiterplatten werden für verschiedenste Messungen und Assays eingesetzt, bei denen auch häufig die Fluoreszenz von Proben gemessen wird. Durch das Vorsehen des Substrats in den Wells von Mikrotiterplatten kann die Fluoreszenzausbeute der Proben signifikant gesteigert werden. Die Substrate können mit verschiedensten Methoden in den Wells eingebracht und fixiert werden. Vorzugsweise werden die Substrate dabei mittels Kleben, Schweißtechniken (z.B. Laserschweißen) und thermisches Verfügen in den Wells fixiert.

Gemäß einer besonders bevorzugten Ausführungsform der vorliegenden Erfindung umfasst oder besteht der feste Träger aus einem Cyclo-Olefin-Copolymer oder Cyclo-Olefin-Polymer und ist Teil einer Mikrotiterplatte bzw. Teil der Wells einer Mikrotiterplatte. Als besonders geeignet hat sich dabei COP 1060R (ZeonorⓇ 1060R) erwiesen. Der Träger ist dabei vorzugsweise mit 10 bis 60 nm, bevorzugt mit bis 40 nm, Metall (z.B. Silber) beschichtet.

Bestimmte Messungen mit fluoreszierenden Substanzen wie Fluorophore werden in Kapillaren durchgeführt. Daher ist es bevorzugt die Substrate in Kapillaren vorzusehen. Eine beispielhafte Anwendung ist die Zytometrie bzw. die Durchflusszytometrie, bei der die Anzahl oder auch die Art fluoreszierender Zellen oder fluoreszenzmarkierter Zellen mit Hilfe einer Fluoreszenzmessung bestimmt wird.

Zahlreiche Anwendungen zur Fluoreszenzmessung erfolgen in mikrofluidischen Chips (z.B. als "Lab-on-a-chip"-Anwendung), wobei im Detektionsbereich solcher Chips die erfindungsgemäßen Substrate vorgesehen sein können.

Auch in herkömmlichen Küvetten können die erfindungsgemäßen Substrate vorgesehen sein. Dadurch kann bei Fluoreszenzmessungen die Fluoreszenzausbeute ebenfalls signifikant gesteigert werden, so dass geringsten Mengen an fluoreszierenden Stoffen in einer Probe gemessen werden können. Erfindungsgemäß kann jede Küvettenform verwendet werden.

Auch bei Teststreifen Systemen ("Lateral Flow Assays"), die für Schnelltestungen oder In-Feld Testungen (Point of Care) eingesetzt werden, können die erfindungsgemäßen Substrate (z.B. im Detektionsbereich ("Detection Line") eingesetzt werden, um die Fluoreszenz eines markierten Analyten (z.B. eines fluoreszenzmarkierten Antikörpers) zu verstärken und so die Empfindlichkeit des Tests zu verbessern.

In eine weiteren bevorzugten Ausführungsform der Erfindung werden die Substrate auf Objektträger wie sie in der Mikroskopie insbesondere der Fluoreszenzmikroskopie eingesetzt werden, aufgebracht. Die Fluoreszenz von zur Markierung zellulärer Strukturen eingesetzter Fluorophore könnte damit selektiv verstärkt und die optische Auflösung der Methoden drastisch verbessert werden, da weniger Lichtintensität benötigt wird, was das Signal/Rausch-Verhältnis optimieren würde. Anwendungsbereiche wären hochauflösende Verfahren wie die konfokale Lasermikroskopie nach dem Punktscanner-Prinzip sowie 4Pi-Mikroskope und STED (Stimulated Emission Depletion)-Mikroskope.

Gemäß einer weiteren bevorzugten Ausführungsform der vorliegenden Erfindung umfasst die Metallbeschichtung an der Oberfläche des Substrats zumindest teilweise Moleküle zur direkten und/oder indirekten Bindung von fluoreszierenden Molekülen.

Die Substrate können die Fluoreszenz von fluoreszierenden Molekülen bzw. von Fluorophoren verstärken, wenn sich die Fluorophore in räumlicher Nähe (vorzugsweise weniger als 20 nm) der Substrate befinden. Die Fluorophore bzw. die fluoreszierenden Substanzen können sich dabei frei in einer Flüssigkeit bewegen, wobei die Fluoreszenzsteigerung nur dann zustande kommt, wenn sich diese Fluorophore bzw. fluoreszierenden Moleküle dem Substrat nähern. Um die Wahrscheinlichkeit der Annäherung der Fluorophore bzw. der fluoreszierenden Moleküle an das Substrat zu erhöhen, ist es von besonderem Vorteil, wenn an der Oberfläche des Substrats (d.h. an der Metallbeschichtung) Moleküle irreversibel oder reversibel gebunden sind, die entweder das Fluorophor bzw. das fluoreszierende Molekül selbst ("direkte Bindung") oder ein Molekül, an welchem ein Fluorophor oder ein fluoreszierendes Molekül (z.B. fluoreszenzmarkierter Antikörper; "indirekte Bindung") gekoppelt ist, binden können. Verfahren zur Bindung von derartigen Molekülen an Metallstrukturen sind hinreichend bekannt. Im einfachsten Fall erfolgt die Bindung durch physikalisch chemische Adsorption (vermittelt über ionische und hydrophobe Wechselwirkungen) der Proteine an die Metalloberfläche (z.B. Nakanishi K. et al. J Biosci Bioengin 91(2001):233-244). Auch kovalente Methoden zur Immobilisierung von Proteinen nach Derivatisierung der Metall-Oberflächen sind bekannt (z.B. GB Sigal et al. Anal Chem 68(1996):490-7).

Moleküle zur direkten und/oder indirekten Bindung von fluoreszierenden Molekülen bzw. Fluorophoren sind vorzugsweise ausgewählt aus der Gruppe bestehend aus Antikörper, Antikörperfragmente, vorzugsweise Fab, F(ab)'2 oder scFv Fragmente, Nukleinsäuren, Enzymen, Lipiden, Viruspartikeln, Aptameren und Kombinationen davon.

Diese Moleküle sind einerseits in der Lage Fluorophore bzw. fluoreszierende Moleküle direkt zu binden (z.B. Antikörper und Fragmente davon, Nukleinsäuren, Enzyme), andererseits können diese Moleküle auch andere Moleküle binden, die mit einem Fluorophor bzw. einer fluoreszierender Substanz versehen sind.

"Fluoreszenzsubstrat für ein Enzym", wie hierin verwendet, ist ein Substrat, welches in der Lage ist in oder am aktiven Zentrum des Enzyms zu binden, wodurch das Substrat fluoreszierende Eigenschaften erlangen kann. Selbstverständlich kann das Substrat auch vor dessen Anlagerung am Enzym fluoreszierende Eigenschaften aufweisen.

Die festen Träger mit den Vertiefungen wie oben definiert werden anschließend mit einem oder mehreren Metallen (z.B. zwei, drei, vier oder fünf Metallen) beschichtet. Verfahren zum Beschichten von festen Trägern mit Metallen sind in der Fachwelt hinreichend bekannt, wobei besonders bevorzugt PVD-Verfahren (PVD; "Physical Vapor Deposition) wie Sputter- oder Aufdampfverfahren eingesetzt werden. Das mindestens eine Metall kann mittels eines Sputterverfahrens oder thermisches Verdampfen, mit Elektronenstrahlverdampfen, Laserstrahlverdampfen, Lichtbogenverdampfen, Molekularstrahlepitaxie, Ionenstrahl gestützte Deposition oder mit Ionenplattieren oder einem sonstigen Verfahren nach dem jeweiligen aktuellen Stand der Technik auf die Oberfläche des festen Trägers aufgebracht.

Um die direkte und/oder indirekte Bindung von Fluorophoren oder sonstigen fluoreszierenden Substanzen auf der Oberfläche des Substrats zu ermöglichen, werden auf der Metallbeschichtung an der Oberfläche des Substrats zumindest teilweise Moleküle zur direkten und/oder indirekten Bindung von Fluorophoren über Adsorptive oder kovalente chemische Derivatisierung der Oberfläche aufgebracht .

"Zumindest teilweise", wie hier verwendet, bedeutet, dass mindestens 10%, vorzugsweise mindestens 30%, noch mehr bevorzugt mindestens 50%, noch mehr bevorzugt mindestens 70%, noch mehr bevorzugt mindestens 90%, noch mehr bevorzugt mindestens 90%, insbesondere 100%, des mit Metall beschichteten festen Trägers mit Moleküle zur direkten und/oder indirekten Bindung von Fluorophoren versehen ist.

Gemäß einer bevorzugten Ausführungsform der vorliegenden Erfindung sind die Moleküle zur direkten und/oder indirekten Bindung von Fluorophoren ausgewählt aus der Gruppe bestehend aus Antikörper, Antikörperfragmente, vorzugsweise Fab, F(ab)'2 oder scFv Fragmente, Nukleinsäuren, Enzymen, Lipiden, Viruspartikeln Aptameren und Kombinationen davon.

Ein weiterer Aspekt der vorliegenden Erfindung betrifft ein Verfahren zur Bestimmung oder zur Quantifizierung mindestens eines Analyten in einer Probe umfassend die Schritte:
a) optionales direktes oder indirektes Markieren mindestens einen Analyten mit mindestens einem Fluorophor,
b) Aufbringen von mindestens einen markierten Analyten aus Schritt a) oder eines fluoreszierenden Analyten auf ein Substrat gemäß der vorliegenden Erfindung,
c) Anregen von mindestens einem Fluorophors durch Bestrahlen des Substrats mit Licht geeigneter Wellenlänge, und
d) Messen der Fluoreszenz zur Bestimmung des Vorhandenseins von mindestens einen Analyten in der Probe.

Das Substrat, das in der Lage ist die Fluoreszenzausbeute von Fluorophoren und sonstigen fluoreszierenden Molekülen oder Substanzen signifikant zu erhöhen, kann für Verfahren eingesetzt werden, in denen die Fluoreszenz von Proben gemessen werden soll. Durch die erfindungsgemäße Verwendung des Substrats in solchen Verfahren, lässt sich die Sensitivität solcher Verfahren signifikant erhöhen, so dass nicht nur das Vorhandensein geringster Mengen an zu bestimmenden Analyten bestimmt sondern auch die Quantifizierung (geringer Mengen) von Analyten exakter durchgeführt werden kann.

In einem ersten Schritt werden die zu bestimmenden bzw. die zu quantifizierenden Analyten in einer Probe direkt oder indirekt mit einem Fluorophor oder einer fluoreszierenden Substanz markiert. Bei einer direkten Markierung des Analyten wird das mindestens eine Fluorophor oder die mindestens eine fluoreszierende Substanz kovalent oder nicht-kovalent (z.B. durch Wasserstoffbrückenbindung, elektrostatische Bindung, Van-der-Waals-Kräfte, hydrophobe Wechselwirkungen) an den zu bestimmenden bzw. zu quantifizierenden Analyten gebunden. Bei einer indirekten Markierung werden in die Probe fluoreszenzmarkierte Moleküle (z.B. Antikörper oder Fragmente davon) eingebracht, die in der Lage sind an den Analyten zu binden. Dieser erste Verfahrensschritt ist optional, da es zu bestimmende bzw. zu quantifizierende Analyten gibt, die von sich aus bereits in der Lage sind - bei entsprechender Anregung - zu fluoreszieren. Proben, die derartige Analyten umfassen, können direkt oder nach einer Probenaufbereitung auf das Substrat aufgebracht werden (siehe Schritt b) des erfindungsgemäßen Verfahrens).

Nach Aufbringen des mindestens einen markierten Analyten aus Schritt a) oder des fluoreszierenden Analyten auf das Substrat wird das Fluorophor bzw. die fluoreszierende Substanz oder der fluoreszierende Analyt mittels Bestrahlen des Substrats mit kohärentem oder nicht kohärentem Licht (z.B. Laser oder Xenon-Blitz Lampe) geeigneter Wellenlänge zur Fluoreszenzemission angeregt.

"Licht geeigneter Wellenlänge", wie hier verwendet, bedeutet, dass das im erfindungsgemäßen Verfahren verwendete Licht eine Wellenlänge aufweist, die geeignet ist, die Fluoreszenzemission einer Substanz bei in Kontakt treten zu induzieren. Beispielsweise ist Licht mit einer Wellenlänge von 485 geeignet die Fluoreszenzemission von Fluorescein Isothiocyanat (FITC) zu induzieren.

Nach Anregung der fluoreszierenden Substanzen mittels Lichts emittieren diese Substanzen Licht (Fluoreszenz) einer bestimmten Wellenlänge. Dieses emittierte Licht einer definierten Wellenlänge wird gemessen und kann zur Quantifizierung oder zur Bestimmung der Anwesenheit eines Analyten in einer Probe verwendet werden. Das emittierte Licht kann mit Hilfe von einem Detektor (z.B. Photomultiplier) gemessen werden. Zum Einsatz können hierbei handelsübliche Mikrotiterplatten Lesegeräte (Tecan F200pro, BioTek Synergy, Molecular Devices FilterMax oder SpectraMax Reihe etc.), Flachbett Fluorescence Scanner (z.B. Tecan LS-Reloaded, Fluoreszenzmikroskope oder jedes andere proprietäre Analysesystem (Roche COBAS, Abbot AxSYM, Behring Opus Plus), wenn ein entsprechender Fluoreszenzdetektor integriert wird) gelangen.

Gemäß einer bevorzugten Ausführungsform der vorliegenden Erfindung weist das mindestens eine Fluorophor eine Anregungswellenlänge im Bereich von 360 bis 780 nm, vorzugsweise von 490 bis 680 nm, auf.

Gemäß einer weiteren bevorzugten Ausführungsform der vorliegenden Erfindung weist das mindestens eine Fluorophor eine Emissionswellenlänge im Bereich von 410 bis 800 nm, vorzugsweise von 510 bis 710 nm, auf.

Das mindestens eine Fluorophor ist vorzugsweise ausgewählt aus der Gruppe bestehend aus Methoxycoumarin, Aminocoumarin, Cy2, Alexa Fluor 488, Fluorescein Isothiocyanat (FITC), Alexa Fluor 430, Alexa Fluor 532, Cy3, Alexa Fluor 555, 5-TAMRA, Alexa Fluor 546, Phycoerythrin (PE), Tetramethyl Rhodamin Isothiocyanat (TRITC), Cy3.5, Rhodamin, Alexa Fluor 568, Alexa Fluor 594, Alexa Fluor 633, Alexa Fluor 647, Cy5, Alexa Fluor 660, Cy5.5, Alexa Fluor 680 und Cy7, vorzugsweise aus der Gruppe bestehend aus Fluorescein Isothiocyanat (FITC), Cy3, Phycoerythrin (PE), Tetramethyl Rhodamin Isothiocyanat (TRITC), Cy5 und Alexa Fluor 680.

Gemäß einer bevorzugten Ausführungsform der vorliegenden Erfindung erfolgt das indirekte Markieren des Analyten mit mindestens einem Fluorophor mittels eines Fluorophor-markierten und Analyt-bindenden Moleküls erfolgt.

Gemäß einer weiteren bevorzugten Ausführungsform der vorliegenden Erfindung ist das Analyt-bindende Molekül aus der Gruppe bestehend aus Antikörper, Antikörperfragmente, vorzugsweise Fab, F(ab)'2 oder scFv Fragmente, Nukleinsäuren, Enzymen, Lipiden, Viruspartikeln, Aptameren und Kombinationen davon ausgewählt.

Die vorliegende Erfindung wird anhand der folgenden Figuren und Beispiele eingehender erläutert, ohne jedoch auf diese beschränkt zu sein.

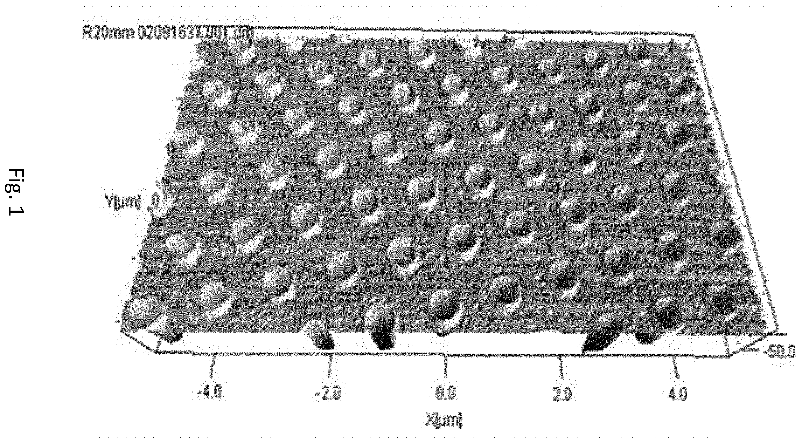

Fig. 1 zeigt eine drei dimensionale AFM ("Atomic Force Microscope"; Rasterkraftmikroskop) Darstellung eines mit Metall beschichteten planaren festen Trägers (siehe Beispiel 1).
Fig. 2 zeigt den MEF-Effekt in Abhängigkeit vom Fluorophortyp und einer Silberschichtdicke von 0, 20 und 50 nm Ag. Der MEF-Effekt äußert sich im beobachteten "relativen Anstieg", also dem Verhältnis des Signals zum Ende der Messzeit nach 600 Sekunden (t600) zum Signal bei Beginn der Messung t(0). Ein relativer Anstieg von 1,0 bedeutet keine Signaländerung und damit keinen MEF. Je höher der relative Anstieg ausfällt, umso stärker ist der MEF-Effekt. Es ist ein genereller Trend zu höherem MEF mit zunehmender Metallschichtdicke zu beobachten, der aber von Fluorophor zu Fluorophor variiert.
Fig. 3 zeigt die Abhängigkeit des MEF von Silberschichtdicke in 5 nm Schritten für AlexaFlour 680 (siehe Beispiel 2). Ab einer Schichtdicke von 5 nm ist eine deutliche Zunahme des MEF-Effekts zu beobachten.
Fig. 4 und 5 zeigen AFM Aufnahmen von Substraten/Strukturen umfassend Vertiefungen unterschiedlicher Periode.
Fig. 6 zeigt die Abhängigkeit des MEF-Effekts von der Periode (0,8 bis 2,2 pm) der Strukturen.
Fig. 7 zeigt die Abhängigkeit des MEF von der Tiefe der Strukturen.
Fig. 8 und 9 zeigen die erhaltenen MEF-Verstärkungsfaktoren im Vergleich zu Kolloid beschichteten Oberflächen und MEF Oberflächen aus dem Stand der Technik (Fa. PLASMONIX; Quanta-Wells 2; "Mitbewerber Struktur").
Fig. 10 zeigt eine MEF-Kinetik auf Nano-Pillars (Nano-Säulen, Erhebungen) und Inverted Nano Pillars (Vertiefungen).
Fig. 11 zeigt die Durchführung eines Anti-Hasen IgG Fluoreszenz Immunoassays unter Verwendung eines Substrates.

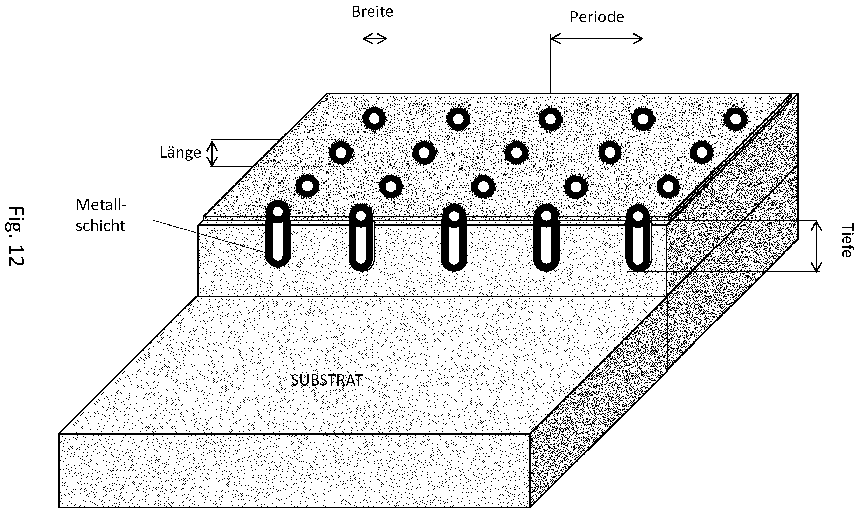

Fig. 12 zeigt das Substrat umfassend einen festen Träger, der mit einer Metallschicht überzogen ist. Der feste Träger weist Vertiefungen mit einer Tiefe, einer Breite und einer Länge auf. Die Vertiefungen befinden sich am festen Träger in einem bestimmten Abstand (Periode) zueinander.

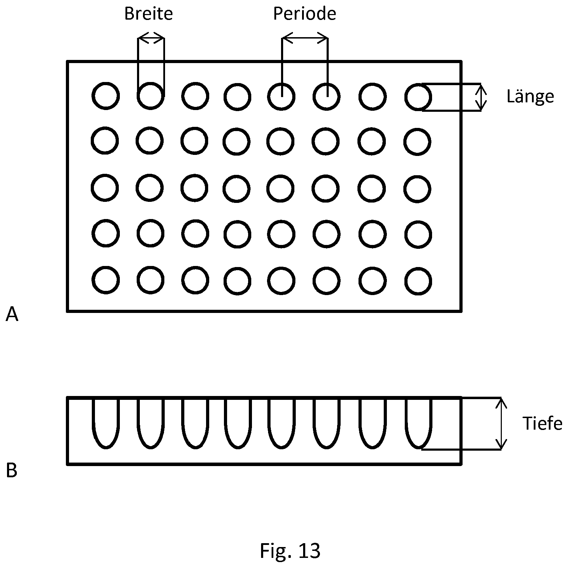

Fig. 13 zeigt die Draufsicht (A) und einen Querschnitt (B) eines festen Trägers. Die Vertiefungen am festen Träger sind durch eine Breite, Länge und Tiefe gekennzeichnet und weisen einen bestimmten Abstand (Periode) zueinander auf.
Fig. 14 zeigt den MEF Effekt bei Verwendung verschiedener Puffer.
Fig. 15 zeigt verschiedene Verfahren mit denen die festen Träger inklusive Vertiefungen hergestellt werden können.

### BEISPIELE:

### Beispiel 1 (nicht teil der Erfindung) : Herstellung des Substrats

Ausgehend vom bekannten Stand der Technik (siehe u.a. Pompa et al. Nature Nanotechnology 1(2006):126-130; Cade et al. Nanotechnology. 15(2009):20(28),US 2009/0262640) wurde versucht, möglichst hohe, schlanke turm- bzw. säulenförmige Strukturen ("Nano Pillars") herzustellen, um durch ein großes Verhältnis (1:2 bis 1:3) von Durchmesser der Basis zur Höhe der Struktur ("Aspect Ratio") zu einer Ausdünnung der Metallschicht beim Bedampfen und so zur Ausbildung der laut Literatur für den MEF-Effekt nötigen Metallinselstrukturen zu kommen. Daher wurden "Pillars" (Säulen; Erhebungen) mit unterschiedlichem Basisdurchmesser (250-550nm) und unterschiedlicher Höhe (250-850nm) hergestellt.

Zur Herstellung der Substrate wurde eine Spezialform des Spritzguss, nämlich Spritzprägen, angewandt. Beim Spritzprägen wird die von thermoplastischer Kunststoffschmelze in ein leicht geöffnetes Werkzeug bei gleichzeitigem Pressvorgang (=Prägen) eingebracht. Der nanostrukturierte Stempel für den Spritzguss wurde mittels Nickel-Galvanik von einem lithografisch hergestellten Silizium-Master abgezogen. Unter Silizium-Master versteht man hier einen mit Positivlack beschichteten Silizium-Wafer, der mittels "Laser Lithografie" belichtet und anschließend entwickelt wurde.

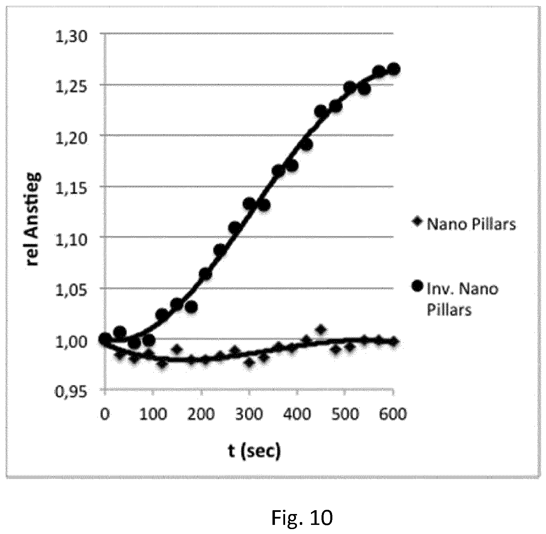

Überraschender Weise zeigten nur die mit Metall beschichteten festen Träger mit den Vertiefungen (INPs) einen deutlichen MEF-Effekt, wohingegen die Substrate basierend auf einem festen Träger mit Erhebungen keinen oder nur einen minimalen MEF-Effekt zeigten (siehe Fig. 10). Daher wurden die INP Strukturen weiter untersucht.

### Beispiel 2: Einfluss der Metall-Schichtdicke

Um zu untersuchen, welchen Einfluss die Metallschichtdicke auf der Oberfläche eines festen Trägers mit Vertiefungen mit einem Durchmesser von ca. 450 nm hat, wurden unterschiedliche Schichtdicken aus Silber aufgedampft.

Die direkte Adsorption fluoreszenzmarkierter Antikörper auf einer Oberfläche ist der einfachste Weg um verschieden strukturierte Oberflächen hinsichtlich Empfindlichkeit und Verstärkungsfaktor zu vergleichen. Der MEF-Effekt zeigte sich dabei darin, dass im Gegensatz zu einer Oberfläche ohne MEF die Bindungskinetik ("MEF-Kinetik") des Antikörpers direkt in Echtzeit verfolgt werden konnte. Dies wurde dadurch ermöglicht, dass nur die Moleküle in der Nähe der Oberfläche stärker leuchten, nicht jedoch die weiter entfernten, ungebundenen Moleküle. Die Lösung mit dem fluoreszenzmarkierten Antikörper wurde dabei auf die entsprechende nano-strukturierte Oberfläche aufgetropft und die Veränderung der Signals über die Zeit mit einem geeigneten Fluoreszentmessgerät (Tecan 200F pro) verfolgt.

Abgesehen vom Parameter "MEF-Kinetik" lässt sich durch den Vergleich des Signals einer bestimmten Konzentration fluoreszenzmarkierten Antikörpers auf einer Oberfläche mit Nano-Metall Struktur mit dem Signal des gleichen Antikörpers auf einer Oberfläche ohne diese Struktur ein Verstärkungsfaktor definieren. Sicherzustellen ist dabei nur, dass die effektiven Belegungsdichten, also die tatsächliche Menge Antikörper auf den der Oberflächen gleich ist.

Dies kann leicht durch Detektion des gebundenen Antikörpers (Goat Anti-Rabbit FITC) mit einem markierten Sekundärantikörper (ein mit alkalischer Phosphatase markierter Donkey Anti-Goat Antikörper) erfolgen und erbrachte keine signifikanten Unterschiede in den Antikörperbelegungsdichten der getesteten Oberflächen.

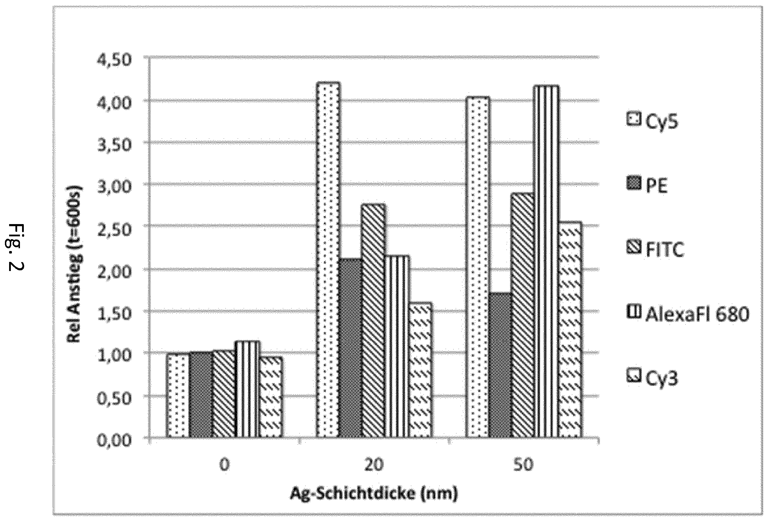

Im Falle der hergestellten Metallschichtdickenvarianten zeigte sich nun, dass der MEF-Effekt im Bereich von 0-50 nm Ag unabhängig vom getesteten Fluorophor signifikant steigt (siehe Fig. 2; Rel. Anstieg von 1 bedeutet kein MEF-Effekt).

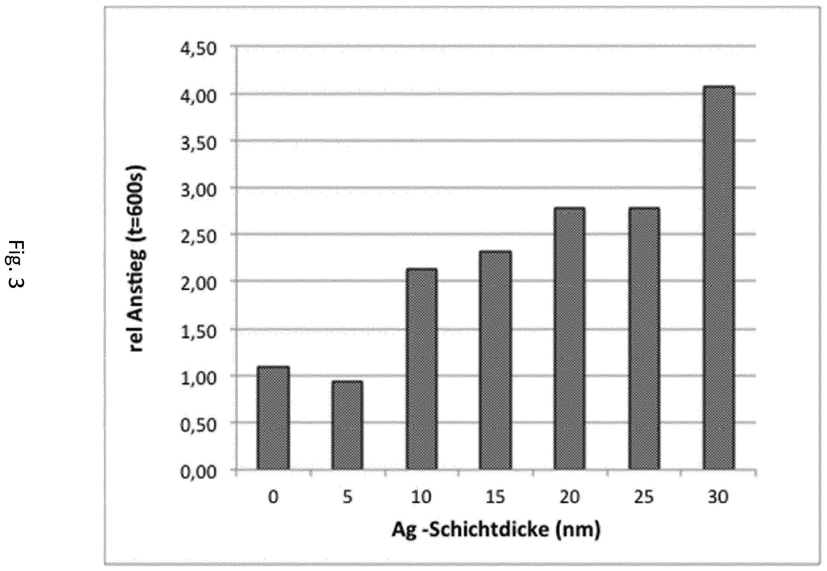

Fig. 3 zeigt, dass eine Mindest-Schichtdicke von 5 nm nötig ist, um einen MEF zu erhalten. Fig. 3 zeigt zudem, dass bei einer Erhöhung der Metallschichtdicke in 5 nm Schritten ein kontinuierlicher Anstieg des MEF-Effekts zu beobachten ist.

### Beispiel 3: Einfluss der Struktur-Periode

Der Abstand der Vertiefungen zueinander ("Periode") könnte einen Einfluss auf den MEF-Effekt des erfindungsgemäßen Substrats haben. Daher wurden verschiedene feste Träger mit unterschiedlichen Perioden beispielhaft mit Silber beschichtet:

| **Feld** | **Periode [µm]** |
|---|---|
| 1 | 0,8 |
| 2 | 1, 0 |
| 3 | 1, 4 |
| 4 | 1, 6 |
| 5 | 1, 8 |
| 6 | 2, 0 |
| 7 | 2, 2 |

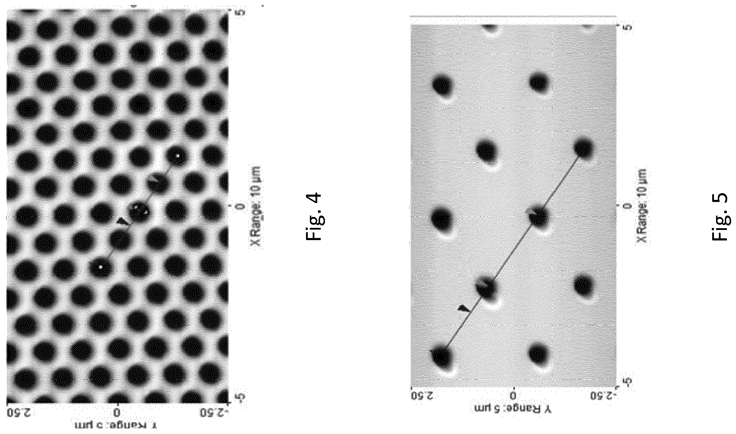

Figuren 4 und 5 zeigen je eine AFM Aufnahme zweier erfindungsgemäßer Substrate mit einer Periode von 0,8 pm bzw. 2,2 pm und einer Silberschichtdicke von 50 nm.

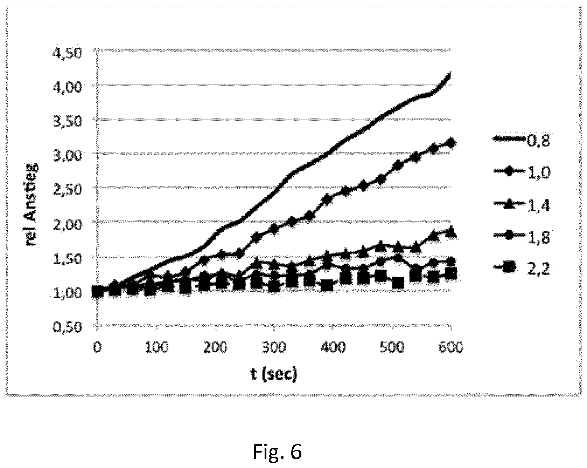

Um den MEF-Effekt nachzuweisen wurden für sämtliche Felder 1 bis 7 MEF-Kinetiken von AlexaFlour 680 (13nM in 10mM PBS, pH 7,4) erstellt (siehe Fig. 6). Dabei konnte festgestellt werden, dass bei einer Periode von 0,8 und 1,0 pm der MEF-Effekt am größten war. Ab einer Periode von 1,2 um war der MEF-Effekt zwar deutlich geringer, aber dennoch vorhanden.

Die nachfolgende Tabelle gibt die relativen Anstiege (Signal t=300s / Signal t=0s) der MEF-Kinetik Messungen verschiedener fluoreszenzmarkierter Antikörper für Feld 1 (0,8um) und 2 (1,0) wieder. Die Silberschichtdicke auf den INPs betrug für diese Messungen 20nm, wobei ein mit dem jeweiligen Fluorophor markierter Ziegenantikörper gerichtet gegen Kaninchen IgG (verdünnt in 10mM PBS pH 7,4; c =13nM) verwendet wurde:

| | **Feld 1** | **Feld 2** |
|---|---|---|
| **FITC** | 1,6 | 1,6 |
| **Cy5** | 1,9 | 2,1 |
| **TRITC** | 1,8 | 1,5 |
| **PE** | 1,3 | 1,3 |
| **Cy3** | 2,3 | 3,2 |
| **AlexaFI680** | 1,7 | 1,7 |

Der MEF-Effekt auf den INPs konnte somit für verschiedenste Fluorophore im Wellenlängenbereich Ex/Em von 485/520 (FITC) bis 680/720 (AlexaFlour 680) demonstriert werden. Die Anwendung der INPs ist nicht auf spezielle Fluorophore beschränkt.

### Beispiel 4: Einfluss der Tiefe der Vertiefungen auf den MEF-Effekt

Um den Einfluss der Tiefe der Vertiefungen (Inverted Nano-Pillars; INPs) zu untersuchen, wurden feste Träger mit unterschiedlichen Vertiefungstiefen (60 nm, 240 nm, 550 nm, 755 nm und 874 nm) hergestellt und mit Silber bedampft (20 nm Schichtdicke) .

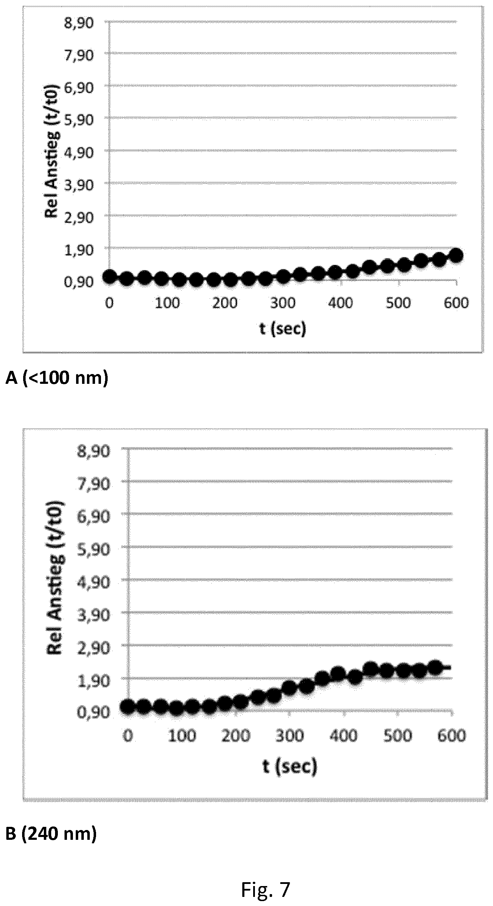

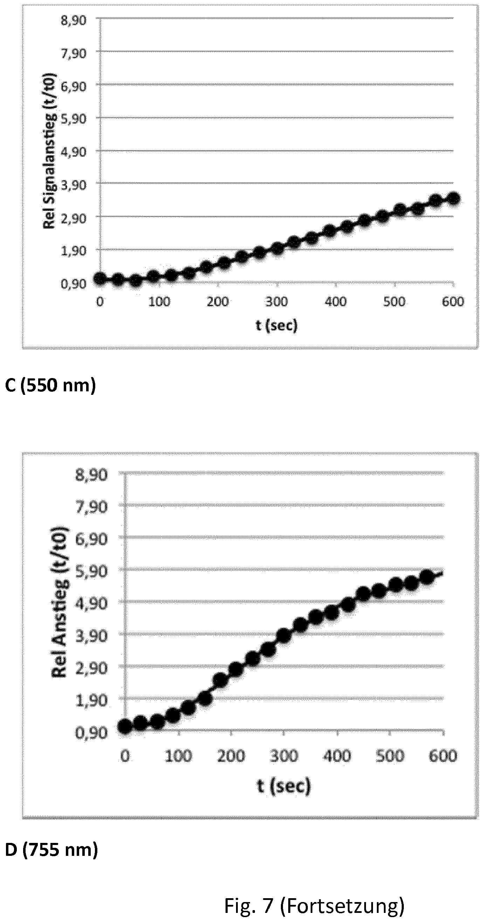

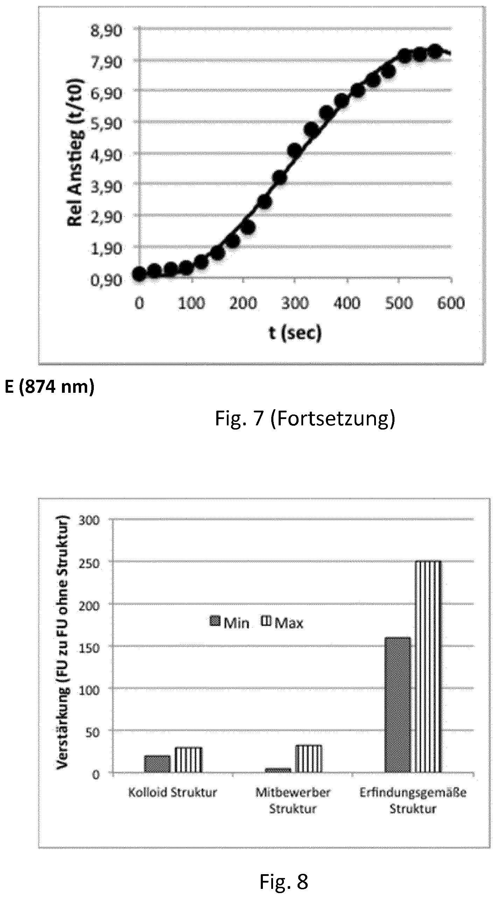

Adsorptionstests mit fluoreszenzmarkierten Antikörpern ("MEF-Kinetik") zeigten, dass der MEF-Effekt mit steigender Vertiefungstiefe ebenfalls ansteigt. Bei festen Trägern mit Vertiefungen von weniger als 60 nm Tiefe wurde ein MEF-Effekt festgestellt, jedoch war dieser deutlich geringer im Vergleich zu den anderen Trägern (siehe Fig. 7).

### Beispiel 5: Vergleichsversuche

Die erfindungsgemäßen Substrate zeigen im Vergleich mit herkömmlich verwendeten Strukturen einen verstärkten MEF-Effekt. Um dies zu belegen, wurden Mikrotiterplatten nach einem aus der Literatur bekannten Verfahren (Direct monitoring of molecular recognition processes using fluorescence enhancement at colloidcoated microplates., C Lobmaier et al Jul 2001; 14(4): 215-22) mit Silber-Kolloiden beschichtet und deren Verstärkungsfaktoren (definiert als Verhältnis der Signale auf Oberfläche ohne und mit Silberkolloiden bei gleicher Antikörper-Oberflächenkonzentration) im Vergleich zu den erfindungsgemäßen Strukturen mit Vertiefungen (20nm Ag, 0.8 pm Periode) ermittelt. Zusätzlich wurde das einzige laut Herstellerangaben auf MEF beruhende kommerzielle Mikrotiterplatten System (Fa. PLASMONIX; Quant-Wells 2) untersucht.

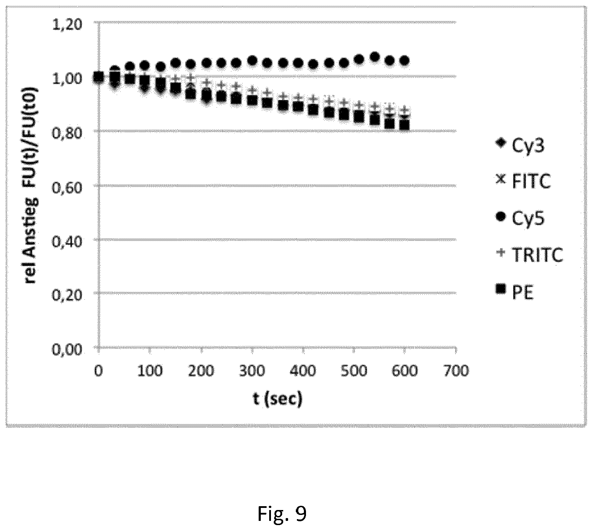

Die Verstärkungsfaktoren des erfindungsgemäßen Substrats lagen, wie in Fig. 9 gezeigt, rund 10x so hoch wie auf Kolloid-Platten oder auf Platten von PLASMONIX. Abgesehen von den deutlich geringeren Verstärkungsfaktoren zeigen die Mikrotiterplatten von PLASMONIX auch nicht die typische MEF-Kinetik (siehe Fig. 9 im Vergleich zu Fig. 7).

### Beispiel 6: Anti-Hasen IgG Fluoreszenz-Immunoassay

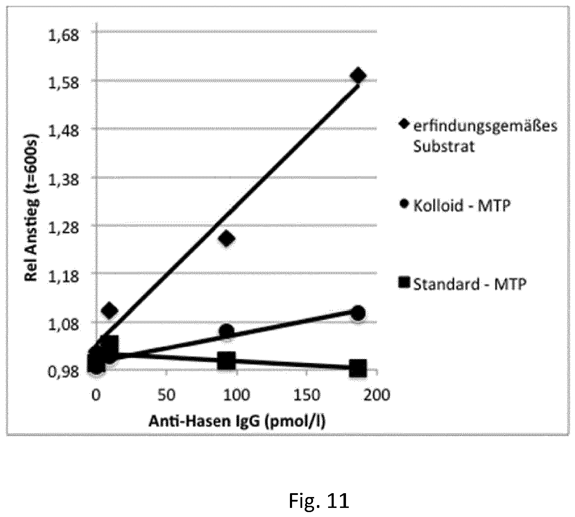

Die Oberflächen eines erfindungsgemäßes Substrats, einer Kolloid beschichtete Mikrotiterplatte (MTP) und einer Standard-Mikrotiterplatte der Fa. Greiner wie sie dem Stand der Technik gemäß für Immunoassays eingesetzt wird, wurden mit einer Lösung von Hasen IgG (2pg/ml) in PBS (10mM Phosphatpuffer mit 150mM NaCl pH 7,4) für 2h bei Raumtemperatur in Kontakt gebracht. Danach wurde die Lösung entfernt, die Oberfläche mit PBS der 0,1% Triton X-100 enthielt gewaschen und mit einer 5% Polyviniylpyrolidon Lösung zur Blockierung unspezifischer Bindungen für 1h in Kontakt gebracht. Nach einem weitern Waschgang mit PBS/Triton X100 erfolgte eine Inkubation mit biotinmarkiertem Anti-Hasen IgG Antikörper verschiedener Konzentration für 1h bei RT. Die Bindung dieses Anti-Hasen IgG Antikörpers wurde schließlich nach einem finalen Waschgang durch eine MEF-Kinetik Messung mit Cy3-markiertem Streptavidin über einen Zeitraum von 600 Sekunden nachgewiesen (siehe Fig 11). Es ist deutlich zu erkennen, dass auf der Standard-Mikrotiterplatte keine MEF-Kinetik auftritt und damit der Immunoassay auch nicht durchführbar ist. Die Kolloid beschichtete Mikrotiterplatte zeigt nur eine schwache, das erfindungsgemäße Substrat hingegen einen sehr deutlich ausgeprägte MEF-Kinetik und damit auch einen Immunoassay mit wesentlich steilerer Eichkurve d.h. wesentlich höherer Empfindlichkeit.

Das in diesem Beispiel verwendete erfindungsgemäße Substrat zeigte vor dessen Beschichtung mit Antikörpern elektrische Leitfähigkeit. Nach erfolgter Messung der MEF Kinetik konnte keine elektrische Leitfähigkeit des Substrats festgestellt werden. Dies könnte durch die Ausbildung von Silberchlorid bei Kontakt mit PBS Puffer bedingt sein.

### Beispiel 7: MEF Effekt in Abhängigkeit des verwendeten Puffers

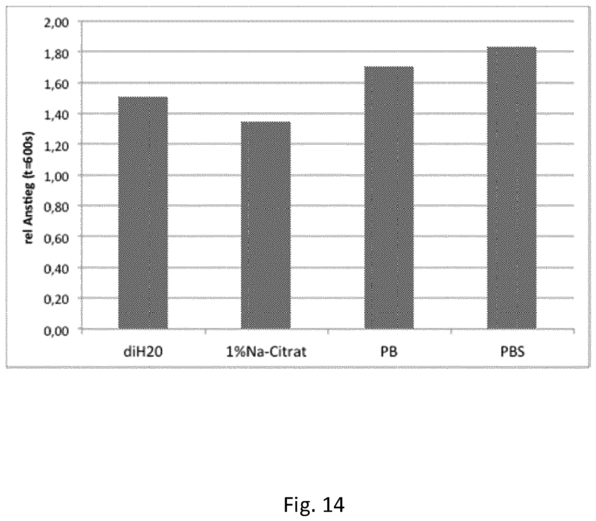

Um die Abhängigkeit des MEF Effekts vom verwendeten Puffer zu untersuchen, wurde wie in Beispiel 3 die durch die Adsorption eines fluoreszenzmarkierten Antikörpers (Ziege Anti-Kaninchen Antikörper, markiert mit Cy5) bedingte MEF-Kinetik beobachtet, wobei anstelle des PBS Puffers ein reiner Phosphatpuffer (PB; 10mM Phosphatpuffer), 1% (w/v) wässrige Natrium-Citrat Lösung und diH₂0 eingesetzt wurde. Durchgeführt wurden die Tests auf Substraten mit einer Periode von 1um (entsprechend Feld 2, siehe Beispiel 3). Wie aus Fig. 14 zu erkennen ist, lieferte die Adsorption aus PBS zwar den höchsten relativen Signalanstieg, aber es waren auch deutliche Signale bei Adsorption des Antikörpers aus den anderen Lösungen zu beobachten. Dies könnte eine Folge der auch in Beispiel 6 beschriebenen, möglichen Ausbildung einer Silberchloridschicht sein, die sich positiv auf den Verstärkungseffekt auswirkt.

## Patentansprüche

1. Verwendung eines Substrats zur Verstärkung der Fluoreszenz eines oder mehrerer fluoreszierenden Moleküle, **dadurch gekennzeichnet, dass** das Substrat einen festen polymeren Träger mit einer Mehrzahl voneinander getrennter Vertiefungen umfasst und der feste Träger zumindest teilweise mit Silber oder Legierungen umfassend Silber beschichtet ist, wobei die Vertiefungen einen Abstand zueinander von 0,2 µm bis 2,5 µm und eine Tiefe von 0,1 µm bis 5 µm aufweisen und die Metallschicht am festen Träger eine Dicke von 10 nm bis 60 nm aufweist.

2. Verwendung nach Anspruch 1, **dadurch gekennzeichnet, dass** die Vertiefungen des festen Trägers eine Länge und eine Breite aufweisen, wobei das Verhältnis der Länge zur Breite 2:1 bis 1:2, insbesondere 1:1, beträgt.

3. Verwendung nach Anspruch 1 oder 2, **dadurch gekennzeichnet, dass** die Vertiefungen des festen Trägers eine Länge und eine Breite aufweisen, wobei die Länge und die Breite der Vertiefungen 0,1 µm bis 2 µm beträgt.

4. Verwendung nach einem der Ansprüche 1 bis 3, **dadurch gekennzeichnet, dass** die Vertiefungen eine im Wesentlichen runde Form aufweisen.

5. Verwendung nach einem der Ansprüche 1 bis 4, **dadurch gekennzeichnet, dass** der feste Träger mindestens ein Material ausgewählt aus der Gruppe bestehend aus der Gruppe der thermoplastischen Polymere und der Polykondensate umfasst.

6. Verwendung nach Anspruch 5, **dadurch gekennzeichnet, dass** das Material des polymeren festen Trägers organische und/oder anorganische Zusatzstoffe und/oder Füllstoffe umfasst, wobei diese vorzugsweise ausgewählt sind aus der Gruppe bestehend aus TiO₂, Glas, Kohlenstoff, Farbpigmente, Lipide und Wachse.

7. Verwendung nach einem der Ansprüche 1 bis 6, **dadurch gekennzeichnet, dass** das Substrat Teil einer Kapillare, einer Mikrotiterplatte, eines mikrofluidischen Chips, eines Teststreifens, eines Trägers für die Fluoreszenzmikroskopie, eines Sensor Arrays oder eines optischen Detektorfeldes ist.

8. Verwendung nach einem der Ansprüche 1 bis 7, **dadurch gekennzeichnet, dass** die Metallbeschichtung an der Oberfläche des Substrats zumindest teilweise Moleküle zur direkten und/oder indirekten Bindung von fluoreszierenden Molekülen umfasst.

9. Verfahren zur Bestimmung oder zur Quantifizierung mindestens eines Analyten in einer Probe umfassend die Schritte:
a) optionales direktes oder indirektes Markieren des mindestens einen Analyten mit mindestens einem Fluorophor,
b) Aufbringen des mindestens einen markierten Analyten aus Schritt a) oder eines fluoreszierenden Analyten auf ein Substrat wie in einem der Ansprüche 1 bis 8 definiert,
c) Anregen des mindestens eines Fluorophors durch Bestrahlen des Substrats mit Licht geeigneter Wellenlänge, und
d) Messen der Fluoreszenz zur Bestimmung des Vorhandenseins oder zur Quantifizierung des mindestens einen Analyten in der Probe.

10. Verfahren nach Anspruch 9, **dadurch gekennzeichnet, dass** das mindestens eine Fluorophor eine Anregungswellenlänge im Bereich von 360 bis 780 nm, vorzugsweise von 490 bis 680 nm, aufweist.

11. Verfahren nach Anspruch 9 oder 10, **dadurch gekennzeichnet, dass** das mindestens eine Fluorophor eine Emissionswellenlänge im Bereich von 410 bis 800 nm, vorzugsweise von 510 bis 710 nm, aufweist.

12. Verfahren nach einem der Ansprüche 9 bis 11, **dadurch gekennzeichnet, dass** das mindestens eine Fluorophor ausgewählt ist aus der Gruppe bestehend aus Methoxycoumarin, Aminocoumarin, Cy2, Alexa Fluor 488, Fluorescein Isothiocyanat (FITC), Alexa Fluor 430, Alexa Fluor 532, Cy3, Alexa Fluor 555, 5-TAMRA, Alexa Fluor 546, Phycoerythrin (PE), Tetramethyl Rhodamin Isothiocyanat (TRITC), Cy3.5, Rhodamin, Alexa Fluor 568, Alexa Fluor 594, Alexa Fluor 633, Alexa Fluor 647, Cy5, Alexa Fluor 660, Cy5.5, Alexa Fluor 680 und Cy7, vorzugsweise aus der Gruppe bestehend aus Fluorescein Isothiocyanat (FITC), Cy3, Phycoerythrin (PE), Tetramethyl Rhodamin Isothiocyanat (TRITC), Cy5 und Alexa Fluor 680.

13. Verfahren nach einem der Ansprüche 9 bis 12, **dadurch gekennzeichnet, dass** das indirekte Markieren des Analyten mit mindestens einem Fluorophor mittels eines Fluorophor-markierten und Analyt-bindenden Moleküls erfolgt.

14. Verfahren nach Anspruch 13, **dadurch gekennzeichnet, dass** das Analyt-bindende Molekül aus der Gruppe bestehend aus Antikörper, Antikörperfragmente, vorzugsweise Fab, F(ab)'2 oder scFv Fragmente, Nukleinsäuren, Enzymen, Lipiden, Viruspartikeln, Aptameren und Kombinationen davon ausgewählt ist.

## Claims

1. Use of a substrate for enhancing the fluorescence of one or several fluorescent molecules, **characterized in that** the substrate comprises a solid polymer carrier having a plurality of recesses separated from each other and that the solid carrier is coated at least in part by silver or alloys comprising silver, wherein the recesses have a distance to each other of 0.2 µm to 2.5 µm and a depth of 0.1 µm to 5 µm and wherein the metal layer on the solid carrier has a thickness of 10 nm to 60 nm.

2. Use according to claim 1, **characterized in that** the recesses of the solid carrier have a length and a width, wherein the ratio of length to width is 2:1 to 1:2, in particular 1:1.

3. Use according to claim 1 or 2, **characterized in that** the recesses of the solid carrier have a length and a width, wherein the length and the width of the recesses are 0.1 µm to 2 µm.

4. Use according to any of claims 1 to 3, **characterized in that** the recesses have an essentially round shape.

5. Use according to any of claims 1 to 4, **characterized in that** the solid carrier comprises at least one material selected from the group consisting of the thermoplastic polymers and of the polycondensates.

6. Use according to claim 5, **characterized in that** the material of the solid polymer carrier comprises organic and/or inorganic additives and/or fillers, wherein these are preferably selected from the group consisting of TiO₂, glass, carbon, colour pigments, lipids and waxes.

7. Use according to any of claims 1 to 6, **characterized in that** the substrate is part of a capillary tube, a microtiter plate, a microfluidic chip, an assay strip, a carrier for fluorescence microscopy, a sensor array or an optical detector field.

8. Use according to any of claims 1 to 7, **characterized in that** the metal coating on the surface of the substrate comprises at least in part molecules for the direct and/or indirect bonding of fluorescent molecules.

9. A method for determining or quantifying at least one analyte in a sample, comprising the steps of:
a) optional direct or indirect labelling of the at least one analyte with at least one fluorophore,
b) applying the at least one labelled analyte from step a) or a fluorescent analyte onto a substrate as defined in any of claims 1 to 8,
c) exciting the at least one fluorophore by irradiation of the substrate using light at an appropriate wavelength, and
d) measuring the fluorescence in order to determine the presence or in order to quantify the at least one analyte in the sample.

10. A method according to claim 9, **characterized in that** the at least one fluorophore has an excitation wavelength in the range of 360 to 780 nm, preferably of 490 to 680 nm.

11. A method according to claim 9 or 10, **characterized in that** the at least one fluorophore has an emission wavelength in the range of 410 to 800 nm, preferably of 510 to 710 nm.

12. A method according to any of claims 9 to 11, **characterized in that** the at least one fluorophore is selected from the group consisting of methoxy coumarine, amino coumarine, Cy2, Alexa Fluor 488, fluorescein isothiocyanate (FITC), Alexa Fluor 430, Alexa Fluor 532, Cy3, Alexa Fluor 555, 5-TAMRA, Alexa Fluor 546, phycoerythrine (PE), tetramethyl rhodamine isothiocyanate (TRITC), Cy3.5, rhodamine, Alexa Fluor 568, Alexa Fluor 594, Alexa Fluor 633, Alexa Fluor 647, Cy5, Alexa Fluor 660, Cy5.5, Alexa Fluor 680 and Cy7, preferably from the group consisting of fluorescein isothiocyanate (FITC), Cy3, phycoerythrine (PE), tetramethyl rhodamine isothiocyanate (TRITC), Cy5 and Alexa Fluor 680.

13. A method according to any of claims 9 to 12, **characterized in that** the indirect labelling of the analyte with at least one fluorophore is carried out by means of a fluorophore-labelled and analyte-binding molecule.

14. A method according to claim 13, **characterized in that** the analyte-binding molecule is selected from the group consisting of antibodies, antibody fragments, preferably Fab, F (ab) '2 or scFv fragments, nucleic acids, enzymes, lipids, virus particles, aptamers and combinations thereof.

## Revendications

1. Utilisation d'un substrat pour l'amplification de la fluorescence d'une ou de plusieurs molécules fluorescentes, **caractérisée en ce que** le substrat comprend un support polymère solide avec une pluralité de cavités séparées les unes des autres et que le support solide est recouvert au moins partiellement avec de l'argent ou des alliages comprenant de l'argent, les cavités présentant une distance entre elles de 0,2 µm à 2,5 |im et une profondeur de 0,1 µm à 5 µm et la couche métallique sur le support solide présentant une épaisseur de 10 nm à 60 nm.

2. Utilisation selon la revendication 1, **caractérisée en ce que** les cavités du support solide présentent une longueur et une largeur, le rapport de la longueur à la largeur étant de 2:1 à 1:2, en particulier de 1:1.

3. Utilisation selon la revendication 1 ou 2, **caractérisée en ce que** les cavités du support solide présentent une longueur et une largeur, la longueur et la largeur des cavités étant de 0,1 µm à 2 µm.

4. Utilisation selon l'une des revendications 1 à 3, **caractérisée en ce que** les cavités présentent une forme sensiblement ronde.

5. Utilisation selon l'une des revendications 1 à 4, **caractérisée en ce que** le support solide comprend au moins un matériau sélectionné dans le groupe constitué du groupe des polymères thermoplastiques et des polycondensats.

6. Utilisation selon la revendication 5, **caractérisée en ce que** le matériau du support solide polymère comprend des additifs et/ou des agents de charge organiques et/ou minéraux, ceux-ci étant de préférence sélectionnés dans le groupe constitué du TiO₂, du verre, du carbone, de pigments colorés, de lipides et de cires.

7. Utilisation selon l'une des revendications 1 à 6, **caractérisée en ce que** le substrat fait partie d'un capillaire, d'une plaque de micro-titrage, d'une puce microfluidique, d'une bandelette de test, d'un support pour la microscopie à fluorescence, d'un réseau de capteurs ou d'un réseau de détecteurs optique.

8. Utilisation selon l'une des revendications 1 à 7, **caractérisée en ce que** le revêtement métallique à la surface du substrat comprend au moins en partie des molécules pour la liaison directe et/ou indirecte de molécules fluorescentes.

9. Procédé pour la détermination ou pour la quantification d'au moins un analyte dans un échantillon comprenant les étapes suivantes :
a) marquage direct ou indirect facultatif de l'au moins un analyte avec au moins un fluorophore,
b) application de l'au moins un analyte marqué provenant de l'étape a) ou d'un analyte fluorescent sur un substrat tel qu'il est défini dans l'une des revendications 1 à 8,
c) excitation de l'au moins un fluorophore par l'irradiation du substrat avec une lumière d'une longueur d'onde appropriée, et
d) mesure de la fluorescence pour la détermination de la présence ou pour la quantification de l'au moins un analyte dans l'échantillon.

10. Procédé selon la revendication 9, **caractérisé en ce que** l'au moins un fluorophore présente une longueur d'onde d'excitation située sur la plage de 360 à 780 nm, de préférence de 490 à 680 nm.

11. Procédé selon la revendication 9 ou 10, **caractérisé en ce que** l'au moins un fluorophore présente une longueur d'onde d'émission située sur la plage de 410 à 800 nm, de préférence de 510 à 710 nm.

12. Procédé selon l'une des revendications 9 à 11, **caractérisé en ce que** l'au moins un fluorophore soit sélectionné dans le groupe constitué de la méthoxycoumarine, de l'aminocoumarine, du Cy2, de l'Alexa Fluor 488, de l'isothiocianate de fluorescéine (FITC), de l'Alexa Fluor 430, de l'Alexa Fluor 532, du Cy3, de l'Alexa Fluor 555, du 5-TAMRA, de l'Alexa Fluor 546, de la phycoérythrine (PE), de l'isothiocyanate de tétraméthyle de rhodamine (TRITC), du Cy3.5, de la rhodamine, de l'Alexa Fluor 568, de l'Alexa Fluor 594, de l'Alexa Fluor 633, de l'Alexa Fluor 647, du Cy5, de l'Alexa Fluor 660, du Cy5.5, de l'Alexa Fluor 680 et du Cy7, de préférence parmi le groupe constitué de l'isothiocianate de fluorescéine (FITC), du Cy3, de la phycoérythrine (PE), de l'isothiocyanate de tétraméthyle de rhodamine (TRITC), du Cy5 et de l'Alexa Fluor 680.

13. Procédé selon l'une des revendications 9 à 12, **caractérisé en ce que** le marquage indirect de l'analyte avec au moins un fluorophore se fait au moyen d'une molécule marquée par le fluorophore et liant l'analyte.

14. Procédé selon la revendication 13, **caractérisé en ce que** la molécule liant l'analyte est sélectionnée dans le groupe constitué d'anticorps, de fragments d'anticorps, de préférence de fragments Fab, F(ab)' 2 ou scFv, d'acides nucléiques, d'enzymes, de lipides, de particules virales, d'aptamères et de combinaisons de ceux-ci.
